# EUROPÄISCHE PATENTSCHRIFT

(11) **EP 1 795 149 B1**
(45) Veröffentlichungstag und Bekanntmachung des Hinweises auf die Patenterteilung: **23.09.2015**
(21) Anmeldenummer: 07000910.5
(22) Anmeldetag: 10.06.2005
(51) Int. Cl.: A61C 13/00, A61C 13/275

(54) **Verfahren und Vorrichtung zur Herstellung von Zahnersatz**
Method and device for producing dentures
Procédé et dispositif destiné à la fabrication de prothèses dentaires

(30) Priorität: 10.06.2004 DE 202004009128 U; 23.06.2004 DE 202004009900 U
(43) Veröffentlichungstag der Anmeldung: 13.06.2007
(62) Teilanmeldung aus: 05759210.7
(73) Patentinhaber: Institut Straumann AG, 4002 Basel (CH)
(72) Erfinder: Weber, Gerhard, 86932 Pürgen (DE); Holzner, Stephan, 80269 Hohenschäftlarn (DE)
(74) Vertreter: Grünecker Patent- und Rechtsanwälte PartG mbB

(56) Entgegenhaltungen:
- WO-A-2004/044787
- JP-A- 5 049 651
- US-A- 6 049 743

## Beschreibung

Die Erfindung betrifft Verfahren und Vorrichtungen zur Herstellung von Zahnersatz insbesondere auf Basis von CAD/CAM-Technologien.

Dokument WO 2004/044787 offenbart ein Verfahren bei dem eine Software so voreingestellt wird, dass eine Form eines Zahnersatzteils automatisch aus einer Zahnbibliothek ausgewählt wird.

### Erweiterung Ponticdatenbank

Bei diesem Aspekt der Erfindung handelt es sich um eine Verbesserung bisher in der Zahntechnik üblicher so genannter Pontics (Zwischenglieder: Diese Konstruktionselemente hängen frei zwischen Pfeilerzähnen und ersetzten funktional einen kompletten fehlenden Zahn) im bzw. zum Einsatz in der CAD/CAM-Technologie für Zahntechnik Bisher wird von der Software oftmals ein in einer Datenbank abgespeichertes Pontic als Zwischenglied vorgeschlagen.

Der Gegenstand der Erfindung ist in Ansprüchen 1 und 8 offenbart.

In der erfindungsgemäß verbesserten Ausführung wird ein der Position des Zahnes entsprechendes Zwischenglied aus einer Datenbank entnommen. Je nach Geschmack des Zahntechnikers können nun verschiedene Ponticformen aus der Datenbank ausgewählt, entnommen und eingesetzt werden.

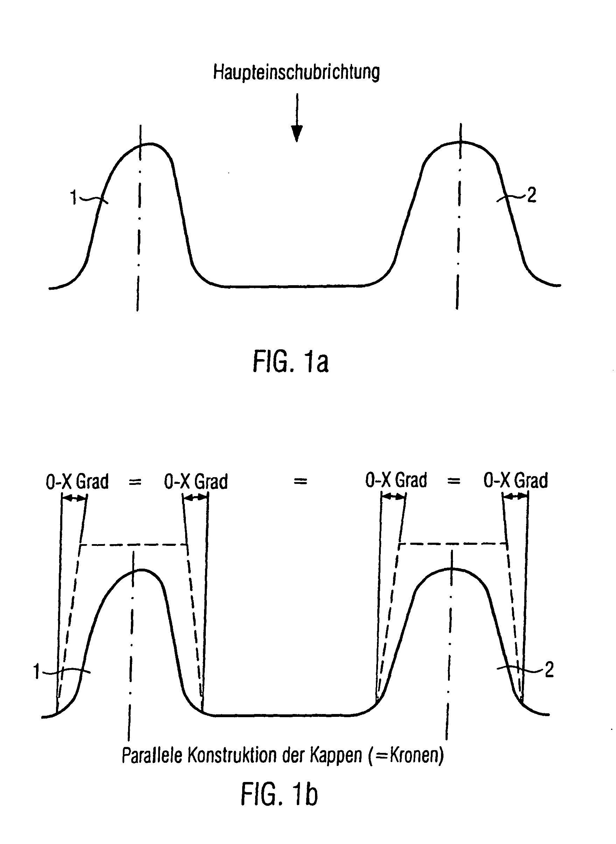

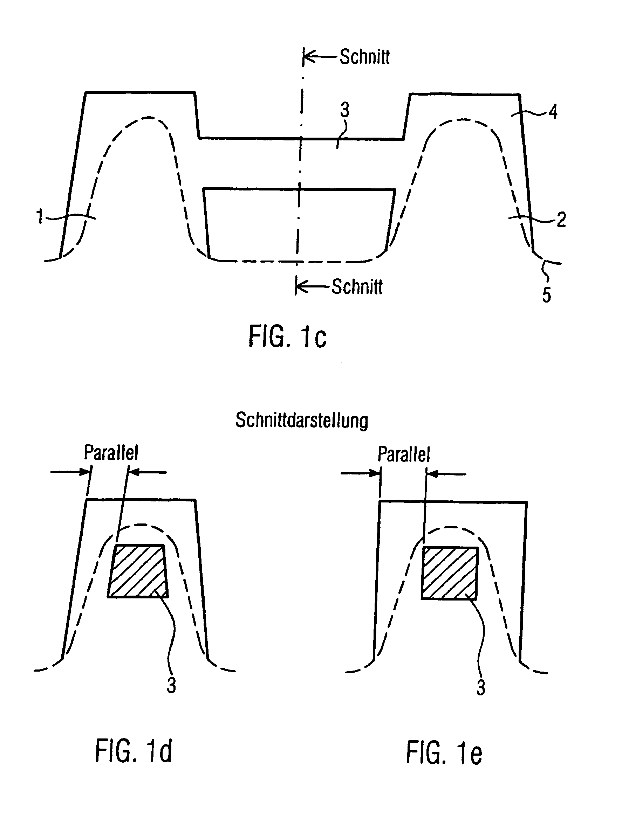

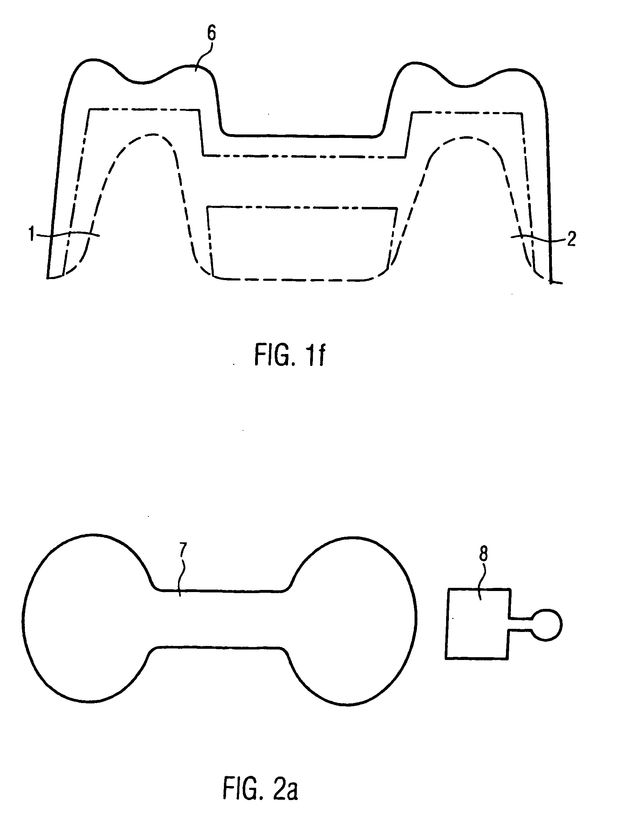

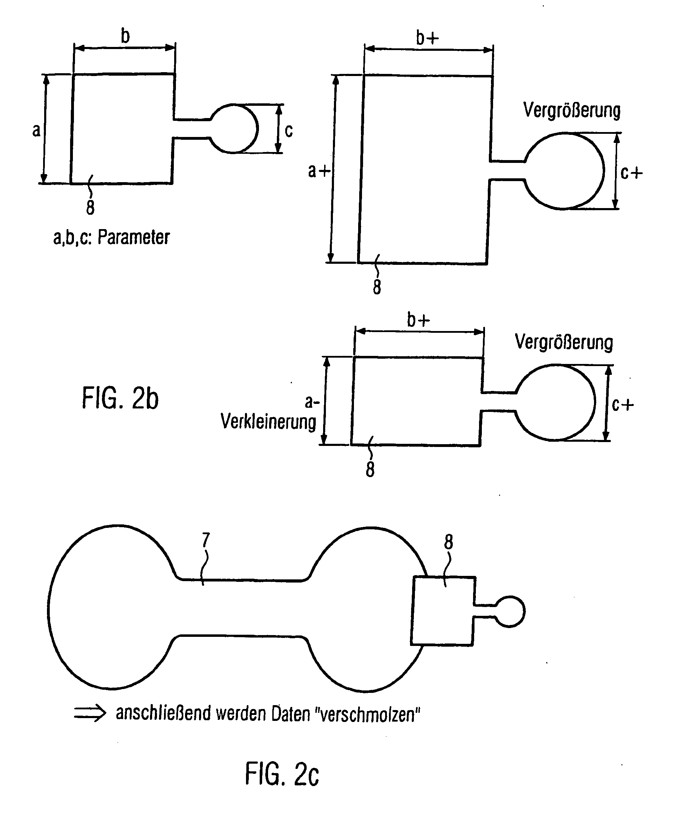

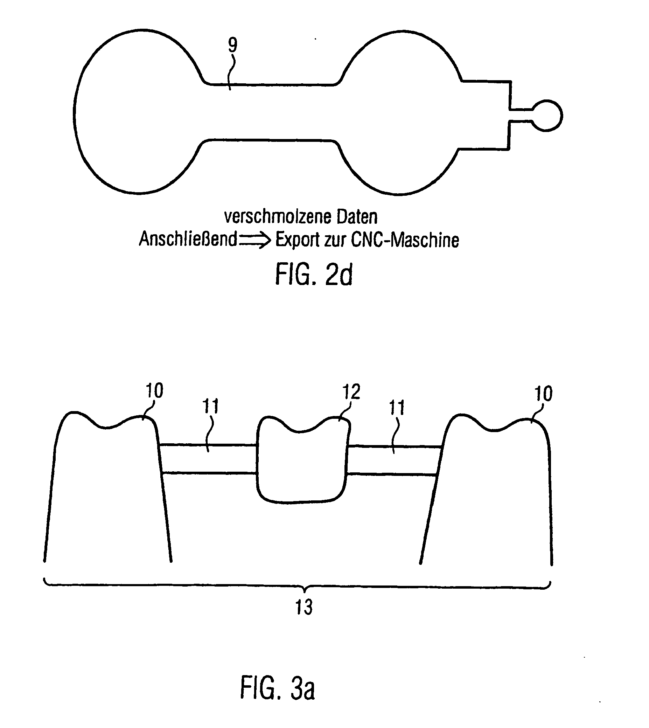

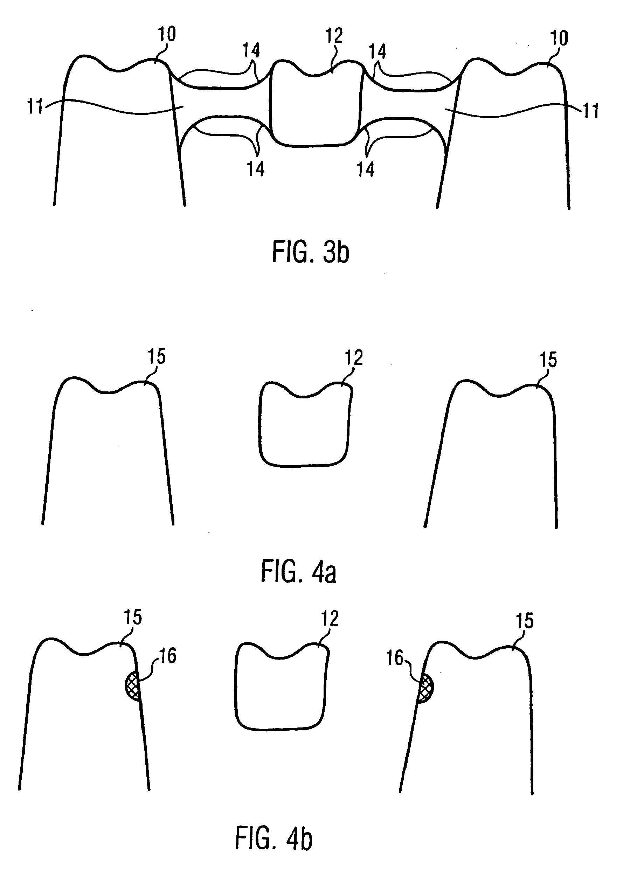

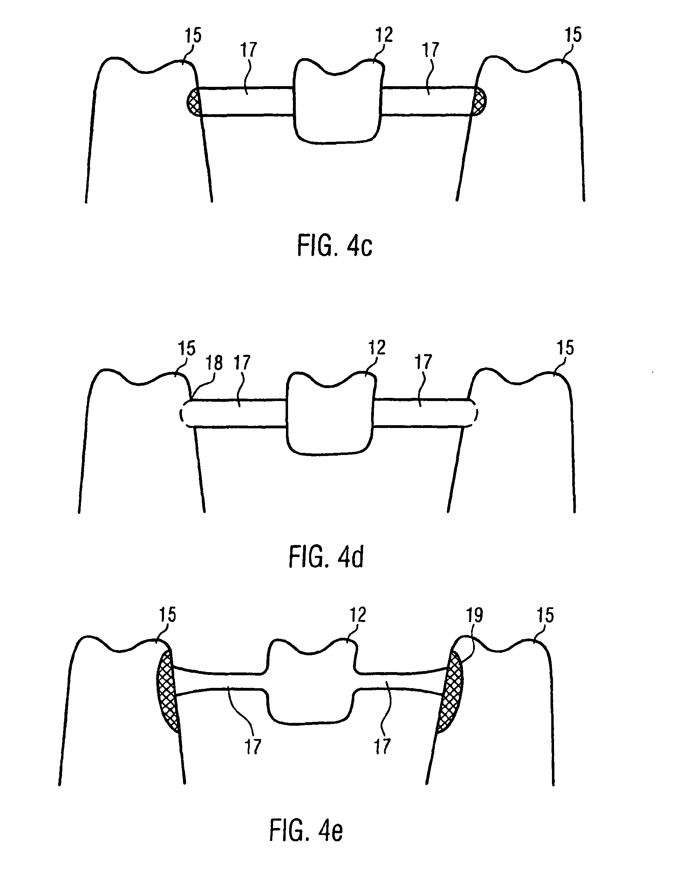

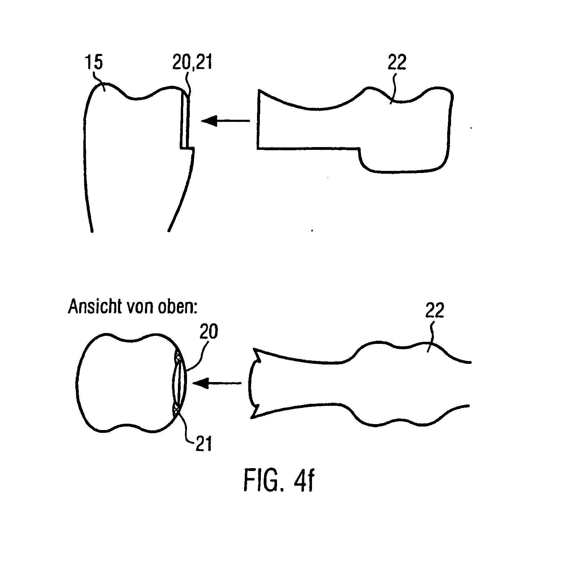

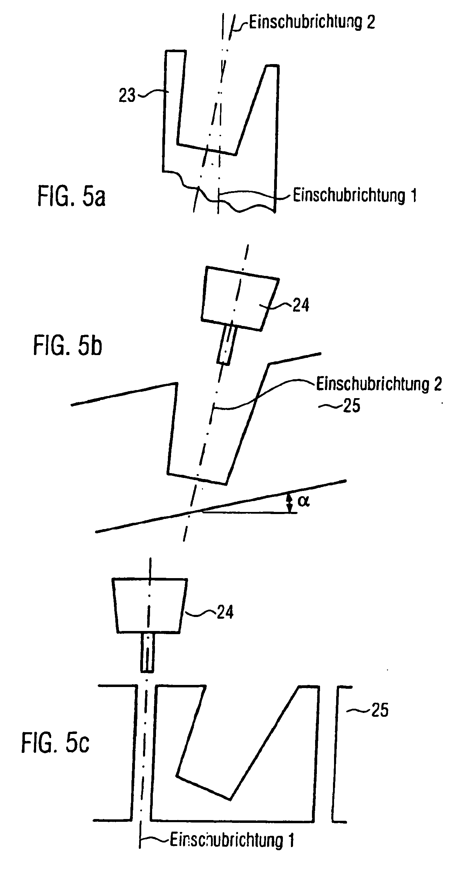

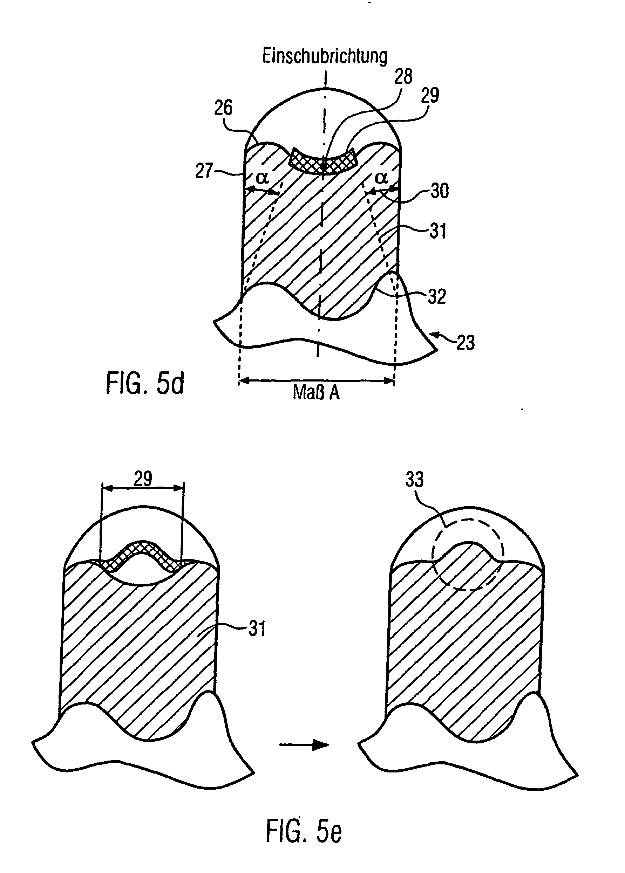

Die Auswahl wird vom Zahntechniker in der Software auch so voreingestellt, dass die von ihm favorisierte Form automatisch als Erstvorschlag für die jeweilige Zahnposition erscheint Weiterhin wird er eine Rangfolge der Favoriten bestimmen und abspeichern. Diese Rangfolge wird nach jedem weiteren Eingabevorgang ersetzt, bis er diesem Vorgang mit einer speziellen Tastenfunktion des Computers, z.B. der "Enter"-Taste bestätigt. Dieselben hier beschriebenen Vorgehensweisen können auch für Verbinder (Fig. 3a, 3b) angewendet werden.

Vorteilhaft sind Vorschläge mit den Grundformen "konvex", "konkav", "plan" im basalen Bereich von Pontics. Ein Aspekt der Erfindung, der für den allgemein bekannten Stand der Technik Gültigkeit hat, besteht in der Möglichkeit, eine individuelle Ponticdatenbank für den Kunden anzubieten. Hierbei entwirft der Zahntechniker mittels CAD-Technik eigene Pontics, die er in einer individuellen Datenbank hinterlegen und abspeichern kann. Bei einer neuen Zahnsituation kann er das selbst entworfene Pontic aus der Kundendatenbank entnehmen, weiterhin geometrisch wandern und anpassen und in den Datensatz für die Brückenkonstruktion eingliedern.

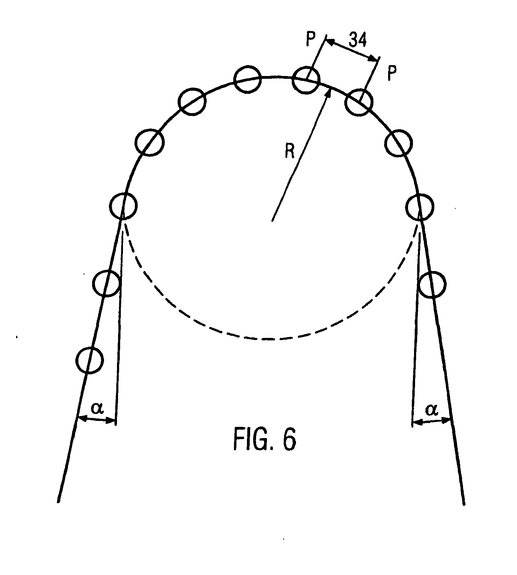

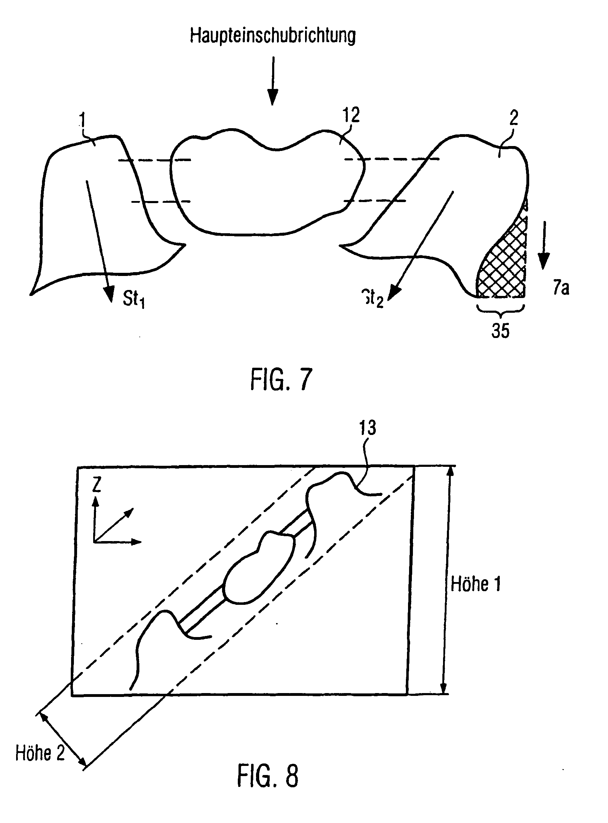

In weiterer Ausgestaltung kann die Bibliothek sozusagen in normierter Größe ausgeführt sein. Wenn ein Zahntechniker ein Pontic auswählt, können über ein Verfahren mit einem Kreis-Tangenten-Modell (vergleiche Figur 6) die Positionen und Skalierungen der Pontics in größeren und kleineren Kiefern aus der programmierten Bibliothek eingefügt werden. Dies erspart einem Anwender sehr viel manuelle Computerarbeit. Lediglich zum besseren Verständnis wird noch angegeben, dass es sich bei dem Kreis-Tangenten-Modell um eine schematisierte Ausführung eines menschlichen Kiefermodells handelt

### "Copy-Cad-Wachs"

Ein sehr verbreitetes Problem ist das Scannen von Modellierwachsen, da sich die teildurchlässige Struktur von Wachs schlecht oder gar nicht scannen lässt. Speziell durch die Zugabe von mindestens 1/3 bis 4/5 Gewichtsanteilen von Zirkonoxidpulver wird das Wachs sehr gut scanbar, ohne dabei die typischen Modelliereigenschaften zu verlieren. Ein solcher Effekt wurde bei der Zugabe von anderen Stoffen nicht beobachtet.

Nachfolgend werden noch weitere Ausführungsmöglichkeiten und Erweiterungen für die einzelnen vorstehend erläuterten Ausführungsbeispiele angegeben.

Um das Arbeiten mit den ermittelten Daten z.B. eines Zahnersatzteils zu erleichtern, kann vorgesehen sein, dass eine farbige Darstellung von Oberflächenbereichen erfolgt. Dazu werden die Daten oder ein daraus erzeugtes elektronisches Bild, wie beispielsweise an einem Computerbildschirm, entweder frei wählbar, manuell oder automatisch anhand von vorgegebenen Kriterien in Oberflächenbereiche eingeteilt. Dann werden diesen Einteilungen und/oder Oberflächenbereichen einzelne Farbgrenzen bzw. Farben zugeordnet und es erfolgt eine entsprechende Darstellung.

So zum Bespiel kann die Innenseite des Zahnersatzteils einem bestimmten Bereich zugeordnet sein. Die Einteilung hat den Vorteil, dass für bestimmte Bereiche spezielle Fertigungsinformationen hinterlegt werden können. Beispielsweise soll auf der Innenseite eines Zahnersatzteiles von einer Maschine genauer gefertigt werden als auf der Außenseite. Besonders präzise soll im Bereich der Präparationsgrenze gearbeitet werden. Hierfür muss z.B. von einer Maschine mit kleinem Werkzeug, hoher Drehzahl und kleinen Vorschüben gearbeitet werden.

Die Einteilung in solche Bereiche wird nach der Erzeugung der Formdatensätze in einem allgemeingültigen Datenformat hinterlegt, das von der Fertigungsart (z.B. Fräsen, Lasersintering, etc.) oder Fertigungsmaschine unabhängig ist. Als Beispiel für ein solches Format könnte ein STL-Format mit Ergilnzungsinformationen verwendet werden. Dieses Format kann an verschiedene weiterverarbeitende Systeme (CAM) übermittelt werden.

Die Erfindung ist anhand der Ausführungsbeispiele in der Beschreibung und in den Zeichnungen lediglich exemplarisch dargestellt.

In den Figuren der Zeichnung bezeichnen:
- 1: Zahnstumpf 1
- 2: Zahnstumpf 2
- 3: Steg
- 4: fräsbares Werkstück
- 5: Zahnsituation (hier gestrichelt)
- 6: abnehmbare Sekundär/Tertiarkonstruktion als Beispiel
- 7: Datensatz Zahnbrücke
- 8: Datensatz geometrisch definierter Zapfen
- 9: verschmolzene Daten aus Datensatz Zahnbrücke und Datensatz geometrisch definierter Zapfen
- 10: Krone
- 11: Verbinder (z.B. stabförmig)
- 12: Pontic
- 13: Brücke
- 14: Verrundungen
- 15: Nachbarzahn
- 16: Markierungen
- 17: Zwischenglied
- 18: Schnittkante
- 19: Verrundung mit größerer Klebefläche
- 20: Rille
- 21: Klebestelle
- 22: Marylandbrücke
- 23: Primärkrone
- 24: Fräser
- 25: Materialrohling
- 26: Friktionslinie oben
- 27: Fläche parallel zur Einschubrichtung
- 28: markierter Punkt
- 29: Wirkungsbereich
- 30: gedachter Konuswinkel α
- 31: glatte Fläche
- 32: Friktionslinie unten
- 33: Ergebnis
- 34: Abstand
- 35: Randschluss

## Patentansprüche

1. Verfahren bei dem eine Software von einem Zahntechniker so voreingestellt wird, dass eine von ihm favorisierte Form eines Pontics automatisch als Erstvorschlag für die jeweilige Zahnposition erscheint, wobei das Verfahren umfasst:
Entnehmen ein der Position des Zahns entsprechendes Pontic aus einer Datenbank, und
Auswählen, Entnehmen und Einsetzen von verschiedenen Ponticformen aus der Datenbank , wobei er eine Rangfolge der Favoriten bestimmt und abspeichert.

2. Verfahren nach Anspruch 1, **dadurch gekennzeichnet, dass** Daten von beispielsweise einem Zahnersatzteil oder ein daraus erzeugtes elektronisches Bild manuell oder automatisch anhand von vorgegebenen Kriterien in Oberflächenbereiche eingeteilt wird und dass dann diesen Einteilungen und/oder Oberflächenbereichen einzelne Farbgrenzen bzw. Farben zugeordnet werden und entsprechend dargestellt werden.

3. Verfahren nach Anspruch 2, **dadurch gekennzeichnet, dass** die Innenseite des Zahnersatzteils einem bestimmten Bereich zugeordnet ist.

4. Verfahren nach Anspruch 2 oder 3, **dadurch gekennzeichnet, dass** für bestimmte Bereiche spezielle Fertigungsinformationen hinterlegt werden.

5. Verfahren nach Anspruch 4, **dadurch gekennzeichnet, dass** auf der Innenseite eines Zahnersatzteils von einer Maschine genauer gefertigt wird als auf der Außenseite.

6. Verfahren nach Anspruch 4 oder 5, **dadurch gekennzeichnet, dass** im Bereich der Präparationsgrenze präziser als in anderen Bereichen gearbeitet wird.

7. Verfahren nach einem der Ansprüche 2 bis 6, **dadurch gekennzeichnet, dass** die Einteilung in Bereiche nach der Erzeugung der Formdatensätze in einem allgemeingültigen Datenformat, wie etwa ein STL-Format mit Ergänzungsinformationen, hinterlegt wird, das von der Fertigungsart oder Fertigungsmaschine unabhängig ist.

8. Vorrichtung zur Herstellung von Zahnersatzteilen mit
Einrichtungen zur digitalen Erfassung von Oberflächendaten eines Restzahnbereichs,
Einrichtungen zur dreidimensionalen Gestaltung wenigstens eines Zahnersatzteils für den Restzahnbereich unter Einbeziehung der erfassten Oberflächendaten des Restzahnbereichs und Erstellung entsprechender Formdaten, und Einrichtungen zur teilweisen oder kompletten Herstellung des wenigstens einen Zahnersatzteiles auf der Basis der Oberflächendaten und Formdaten,
**dadurch gekennzeichnet,**
**dass** Einrichtungen zur Durchführung der Verfahren gemäß jeglichem der Ansprüche 1 bis 7, einschließlich Datenverarbeitungseinrichtungen, Datenspeichereinrichtungen, Bildschirmeinrichtungen, Eingabe- und Ausgabeeinrichtungen sowie Datenübertragungseinrichtungen und Materialbearbeitungseinrichtungen vorgesehen sind, zur Durchführung der Verfahren gemäß jeglichem der Ansprüche 1 bis 7 ausgelegt und funktional miteinander gekoppelt sind.

## Claims

1. Method in which a software is preset such by a dental technician that a form of a pontic favorised by him automatically appears as first proposal for the respective tooth position, wherein the method comprises:
taking a pontic respective to the tooth position from a database and
selecting, taking and inserting of different pontic forms from the database,
wherein he determines and stores a rank order of the favorites.

2. Method according to claim 1 **characterized in that** data for example of a dental restoration or an electronic image generated therefrom is divided manually or automatically in surface regions according to given criteria and that then individual colour borders or colours are assigned to these divisions and/or surface regions and are displayed correspondingly.

3. Method according to claim 2 **characterised in that** the inner side of the dental restoration is assigned to a particular region.

4. Method according to claim 2 and 3 **characterised in that** for particular regions special production information are deposit.

5. Method according to claim 4 **characterised in that** the inner side of a dental restoration is produced more exact by a machine than at the outer side.

6. Method according to claim 4 or 5 **characterised in that** in the region of the preparation line producing is more exact than in other regions.

7. Method according to any one of claims 2 to 6 **characterized in that** the division into regions is deposit after generating the shape data sets in a general data format, like STL format with additional information, which is independent of the production type or production machine.

8. Device for production of dental restorations with
means for digital acquisition of surface data of a remaining dental area,
means for three-dimensional designing of at least one dental restoration for the remaining dental area by taking into account the acquired surface data of the remaining dental area and generating corresponding shape data and means for a partial or complete production of the at least one dental restoration on the basis of the surface data and shape data
**characterised in**
**that** means for performing the methods according to any of the claims 1 to 7 are provided, including data processing means, data storage means, screen means, input and output means as well as data transmission means and material processing means, which are designed and coupled functionally for performing the method according to any of the claims 1 to 7.

## Revendications

1. Procédé selon lequel un logiciel est préréglé par un technicien dentaire de manière à ce qu'apparaisse automatiquement en tant que première suggestion, une forme favorisée par lui, d'un pontique, pour la position de dent respectivement considérée, le procédé comprenant :
le prélèvement dans la banque de données d'un pontique correspondant à la position de la dent, et
la sélection, le prélèvement et la mise en place de différentes formes de pontique de la banque de données,
le technicien définissant et mémorisant un ordre hiérarchique des favoris.

2. Procédé selon la revendication 1, **caractérisé en ce qu'**une série de données relatives par exemple à une pièce de prothèse dentaire ou une image électronique générée à partir de celles-ci, est subdivisée manuellement ou de manière automatique en zones de surface au regard de critères prédéterminés, et **en ce que** des limites de couleur et/ou des couleurs individuelles sont ensuite affectées à ces subdivisions et/ou zones de surface, et sont représentées de manière correspondante.

3. Procédé selon la revendication 2, **caractérisé en ce que** le côté intérieur de la pièce de prothèse dentaire est affecté à une zone déterminée.

4. Procédé selon la revendication 2 ou la revendication 3, **caractérisé en ce que** des informations de fabrication ou d'usinage spéciales ou spécifiques sont consignées pour des zones déterminées.

5. Procédé selon la revendication 4, **caractérisé en ce que** sur le côté intérieur d'une pièce de prothèse dentaire, une machine usine de manière plus précise que sur le côté extérieur.

6. Procédé selon la revendication 4 ou la revendication 5, **caractérisé en ce que** dans la zone de l'interface de préparation, l'usinage de fabrication est plus précis que dans d'autres zones.

7. Procédé selon l'une des revendications 2 à 6, **caractérisé en ce que** la subdivision en zones est consignée, avec des informations complémentaires, après la production des séries de données de forme, dans un format de données valable de manière universelle, comme par exemple un format STL, qui est indépendant du mode de fabrication ou d'usinage, ou de la machine de fabrication ou d'usinage.

8. Installation pour la fabrication de pièces de prothèse dentaire, comprenant
des dispositifs pour l'acquisition numérique de données de surface d'une zone dentaire résiduelle,
des dispositifs pour la configuration tridimensionnelle d'au moins une pièce de prothèse dentaire pour la zone dentaire résiduelle en prenant en considération les données de surface acquises de la zone dentaire résiduelle, et pour l'établissement de données de forme correspondantes, et des dispositifs pour la fabrication partielle ou complète de ladite au moins une pièce de prothèse dentaire sur la base des données de surface et des données de forme,
**caractérisé**
**en ce que** sont prévus des dispositifs configurés pour la mise en oeuvre des procédés selon chacune des revendications 1 à 7, y compris des dispositifs de traitement de données, des dispositifs de mémorisation de données, des dispositifs d'écrans de visualisation, des dispositifs de saisie et de sortie, ainsi que des dispositifs de transmission de données, et des dispositifs d'usinage de matériau configurés pour la mise en oeuvre des procédés selon chacune des revendications 1 à 7, ces dispositifs étant mutuellement couplés sur le plan fonctionnel.
